# EUROPEAN PATENT SPECIFICATION

(11) **EP 3 673 869 B1**
(45) Date of publication and mention of the grant of the patent: **02.07.2025**
(21) Application number: 18849207.8
(22) Date of filing: 01.08.2018
(51) Int. Cl.: A61F 2/24, A61F 2/966

(54) **DELIVERY APPARATUS FOR SELF-EXPANDABLE PROSTHESIS AND DELIVERY APPARATUS FOR SELF-EXPANDABLE HEART VALVE PROSTHESIS**
EINFÜHRUNGSVORRICHTUNG FÜR SELBSTEXPANDIERBARE PROTHESE UND EINFÜHRUNGSVORRICHTUNG FÜR SELBSTEXPANDIERBARE HERZKLAPPENPROTHESE
APPAREIL DE POSE POUR PROTHÈSE AUTO-EXPANSIBLE ET APPAREIL DE POSE POUR PROTHÈSE DE VALVULE CARDIAQUE AUTO-EXPANSIBLE

(30) Priority: 25.08.2017 CN 201710743343
(43) Date of publication of application: 01.07.2020
(73) Proprietor: Shanghai Microport Cardioflow Medtech Co., Ltd., Shanghai 201203 (CN)
(72) Inventor: LI, Longfei, Shanghai 201203 (CN); GUI, Baozhu, Shanghai 201203 (CN); CHEN, Guoming, Shanghai 201203 (CN)
(74) Representative: Lavoix
(86) International application number: PCT/CN2018/097887
(87) International publication number: WO 2019/037579

(56) References cited:
- WO-A1-2007/021708
- CN-A- 103 037 808
- CN-A- 103 655 004
- CN-A- 105 105 870
- CN-A- 105 943 212
- CN-A- 106 175 985
- CN-A- 107 496 055
- US-A1- 2015 265 444
- US-A1- 2015 272 731
- US-A1- 2015 272 731

## Description

### Technical Field

The present application relates to the technical field of medical instrument, and in particular, to a delivery device of a self-expanding prosthesis and a delivery device of a self-expanding heart valve prosthesis.

### Background

Transcatheter aortic valve implantation has advantages of minimal trauma and quick recovery, opening up a survival window for many patients who are terminally ill. The researches on interventional treatment for valvular heart disease markedly accelerate in the new century, and the study on percutaneous valve implantation has been developed from an experimental phase to a small-scale clinical application parallelism phase. The interventional treatment for valvular heart disease is expected to break the technical bottleneck to quickly achieve a wide range of clinical application, and thus has become the focus of attention in the interventional cardiology field. US2015/272731A1 discloses a system for replacing a heart valve of a patient, with an outer primary capsule and a flexible inner secondary capsule.

U.S. 2015/265444A1 discloses a system for replacing a heart valve of a patient. The system includes a delivery device and a prosthetic heart valve. The system is configured to be transitionable between a loaded state, a partially deployed state and a deployed state. In the loaded state, the prosthetic heart valve engages a coupling structure and is compressively retained within a primary capsule, which constrains the prosthetic heart valve in a compressed arrangement. In the partially deployed state, the prosthetic heart valve engages the coupling structure and is compressively retained within a secondary capsule, which constrains the prosthetic heart valve to a partially deployed arrangement. The partially deployed arrangement is less compressed than the compressed arrangement and less expanded than a deployed arrangement. In the deployed state, the primary and secondary capsules are retracted from over the prosthetic heart valve, which expands to the deployed arrangement and is released from the coupling structure.

However, the interventional heart valve replacement at present has many shortcomings in practical clinical application. For example, when an artificial cardiac valve is not completely deployed at the lesion site, the operator finds that the valve deployed position is not ideal and there is a necessity to recapture the valve for relocation and deployment. During recapture of the valve in the human body, a catheter, especially a sheath for compressing and holding the valve, is required to overcome a high resistance without the assistance of an auxiliary instrument such as a loading tool, which requires the sheath has an extremely high tensile property and compressive property. Moreover, the sheath for compressing and holding the valve is required to pass through a high circuitous channel such as the aortic arch, which requires the sheath to have an excellent flexibility. How to balance the two antithetical properties of the sheath has become a difficulty in the art.

It can be known from current clinical application or published reports that, a sheath for compressing and holding a valve in a valve delivery device is mainly made of polymer and metal material. The sheath made of polymer material has an excellent flexibility but a poor resistance to tension and compression, thus failing to recapture the valve for relocation and deployment. The sheath made of metal is used to provide the delivery device with a function of valve recapture. The high resistance to tension and compression generally achieved via the reinforcing ribs on its two sides. Although enhancing the resistance to tension and compression, such a structural design greatly sacrifices the spatial three-dimensional flexibility, that is, the metal sheath can only bend in one plane direction, but is unable to bend in a perpendicular plane direction. However, the aortic arch and the like have a spatial three-dimensionally bending passage. Therefore, such a metal sheath easily punctures the blood vessels when passing through the aortic arch, bringing high risk to the clinical operation.

In view of the deficiencies in the existing valve delivery device, persons skilled in the art are always looking for a solution.

### Summary of the Invention

It is an object of the present application to provide a delivery device of a self-expanding prosthesis to solve the problem that the existing valve delivery device fails to balance functions of valve recapture and valve implantation.

To solve the foregoing technical problem, the present application provides a delivery device according to claim 1.

Optionally, in the delivery device of a self-expanding prosthesis, the internal catheter includes, from a proximal end to a distal end, a first elongated shaft, a fixed head, a second elongated shaft, and a conical head that are successively connected; wherein a proximal end of the first elongated shaft is fixedly connected to the handle, and when delivering the self-expanding prosthesis, the self-expanding prosthesis in the compressed state is loaded by the second elongated shaft, and is detachably connected to the fixed head from a proximal end of the self-expanding prosthesis.

Optionally, in the delivery device of a self-expanding prosthesis, the fixed head includes a main body and at least one protrusion part, each protrusion part being in an elastic connection to the main body.

Optionally, in the delivery device of a self-expanding prosthesis, each protrusion part is connected to the main body via an elastic structure, so as to make the protrusion part capable of performing a telescopic motion along a radial direction of the main body.

Optionally, in the delivery device of a self-expanding prosthesis, a guiding face is provided at a proximal end of each protrusion part to facilitate a smooth passage of the inside external catheter.

Optionally, in the delivery device of a self-expanding prosthesis, the proximal end portion of the inside external catheter includes a third elongated shaft, and the distal end portion of the inside external catheter includes a metal sheath; wherein a proximal end of the third elongated shaft is movably connected to the handle, and at least part of the metal sheath has a hollow structure.

Optionally, in the delivery device of a self-expanding prosthesis, the hollow structure has hollow portions that are in an alternative or whorled arrangement.

Optionally, in the delivery device of a self-expanding prosthesis, the proximal end assembly of the outside external catheter includes a fourth elongated shaft, and the distal end assembly of the outside external catheter includes a polymer sheath; wherein a proximal end of the fourth elongated shaft is connected to the handle and is movable under a driving of the handle.

Optionally, in the delivery device of a self-expanding prosthesis, the handle includes a housing, and a first control mechanism and a second control mechanism that are located in the housing; wherein each of a proximal end of the internal catheter and a proximal end of the stabilizing tube is fixedly connected to the housing; and wherein the first control mechanism is configured to control movements of the inside external catheter, and the second control mechanism is configured to control movements of the outside external catheter.

Optionally, in the delivery device of a self-expanding prosthesis, the housing includes a first housing, a second housing, and a third housing; wherein the first control mechanism is located between the first housing and the second housing, and the second control mechanism is located between the second housing and the third housing; and wherein the proximal end of the internal catheter is fixedly connected to the first housing, and the proximal end of the stabilizing tube is fixedly connected to the third housing.

Optionally, in the delivery device of a self-expanding prosthesis, the first control mechanism includes a first threaded slider, a first threaded knob, and a first locking part; wherein the first threaded knob is restricted between the first housing and the second housing and is able to rotate about an axis of the handle; wherein the first threaded slider, after configured, is fixedly connected to the inside external catheter and is able to move along an axial direction of the handle under a driving of the first threaded knob; and the first locking part is configured to control a rotation of the first threaded knob.

Optionally, in the delivery device of a self-expanding prosthesis, the first threaded knob has a first threaded cavity, into which the first threaded slider is accommodated and is in a threaded connection with the first threaded knob; wherein the first threaded slider is sleeve-jointed and fixed to an outer side of the inside external catheter; and wherein the first locking part is disposed between the first threaded knob and the second housing, and is able to move forward or backward along the axial direction of the handle to drive the first threaded knob to be separated from or connected to the second housing.

Optionally, in the delivery device of a self-expanding prosthesis, the first locking part includes a first button segment, a first snap segment, and a first connection segment that connects the first button segment and the first snap segment; wherein the first button segment is arranged outside the second housing and is able to move along an axial direction of the second housing; wherein the first connection segment extends into an interior of the second housing from an exterior of the second housing; wherein the first snap segment is located on an end surface of the first connection segment close to the first threaded knob, and extends towards the first threaded knob; and wherein an end surface of the first threaded knob adjacent to the first locking part is provided with a first butting groove; and the first snap segment fits into the first butting groove.

Optionally, in the delivery device of a self-expanding prosthesis, the device further includes a pair of guiding mechanisms which are axially parallel with the axial direction of the handle; wherein one end of each guiding mechanism is connected to the first housing, and the other end of each guiding mechanism extends through the first threaded slider and is fixedly connected to the second housing, to restrain the rotation of the first threaded slider.

Optionally, in the delivery device of a self-expanding prosthesis, the second control mechanism includes a second threaded slider, a second threaded knob, and a second locking part; wherein the second threaded knob is limited restricted the second housing and the third housing, and is able to rotate about an axis of the handle; wherein the second threaded slider is configured to be fixedly connected to the outside external catheter, and is able to move along an axial direction of the handle under a driving of the second threaded knob; and wherein the second locking part is configured to limit a rotation of the second threaded knob.

Optionally, in the delivery device of a self-expanding prosthesis, the second threaded knob has a second threaded cavity; wherein the second threaded slider is connected to the outside external catheter after extending through the second threaded cavity, and is provided with an outer thread to form a threaded connection with the second threaded knob; and wherein the second locking part is disposed between the second threaded knob and the second housing, and is able to move forward or backward along the axial direction of the handle to drive the second threaded knob to be separated from or connected to the second housing.

Optionally, in the delivery device of a self-expanding prosthesis, the second locking part includes a second button segment, a second snap segment, and a second connection segment that connects the second button segment and the second snap segment; wherein the second button segment is arranged outside the second housing, and is able to move along an axial direction of the second housing; wherein the second connection segment extends into an interior of the second housing from an exterior of the second housing; wherein the second snap segment is located on an end surface of the second connection segment close to the second threaded knob and extends towards the second threaded knob; and wherein an end surface of the second threaded knob adjacent to the second locking part is provided with a second butting groove; and the second snap segment fits into the second butting groove.

Optionally, in the delivery device of a self-expanding prosthesis, an inner wall of the third housing is provided with rotation limiting grooves that are distributed along an axial direction of the housing, wherein an outer side of the second threaded slider is provided with rotation limiting protrusions that fit into the rotation limiting grooves, and wherein the rotation limiting protrusions are able to move forward or backward along the rotation limiting grooves.

Optionally, in the delivery device of a self-expanding prosthesis, the self-expanding prosthesis is an aortic valve prosthesis, a mitral valve prosthesis, or a tricuspid valve prosthesis.

The present application further provides a delivery device of a self-expanding heart valve prosthesis, including: a catheter and a handle connected to the catheter, wherein the catheter includes an internal catheter, an inside external catheter, an outside external catheter, and a stabilizing tube that are sleeve-jointed one on another in sequence; wherein states of the self-expanding heart valve prosthesis include a compressed state, a semi-deployed state, and a deployed state; wherein the internal catheter is configured to accommodate the self-expanding heart valve prosthesis in the compressed state; wherein the inside external catheter is able to move with respect to the internal catheter, and includes a distal end portion and a proximal end portion, the distal end portion configured to drive the self-expanding heart valve prosthesis to be compressed from the semi-deployed state to the compressed state; wherein the outside external catheter is able to move with respect to the internal catheter, and includes a distal end assembly and a proximal end assembly, the distal end assembly configured to contain the self-expanding heart valve prosthesis to maintain the compressed state of the self-expanding heart valve prosthesis; and wherein the distal end assembly has a better flexibility than the distal end portion, while the distal end portion has a higher tensile property and a higher compressive property than the distal end assembly.

In the delivery device of a self-expanding prosthesis provided in the present application, the delivery device of a self-expanding prosthesis includes a catheter and a handle connected to the catheter. The catheter includes an internal catheter, an inside external catheter, an outside external catheter, and a stabilizing tube that are sleeve-jointed one on another in sequence. The internal catheter is configured to accommodate the self-expanding prosthesis in a compressed state, and a distal end portion of the inside external catheter is configured to drive the self-expanding prosthesis to be compressed from a semi-deployed state to the compressed state. A distal end assembly of the outside external catheter is configured to contain the self-expanding prosthesis to maintain the compressed state of the self-expanding prosthesis. The distal end assembly has a better flexibility than the distal end portion, while the distal end portion has a higher tensile property and a higher compressive property than the distal end assembly. The stabilizing tube and the internal catheter remain stationary relative to each other. The self-expanding prosthesis is able to be deployed by means of the outside external catheter, and the self-expanding prosthesis in the semi-deployed state is able to be recompressed and recaptured to facilitate the deployment again of prosthesis by means of the inside external catheter. Therefore, the single device of the present application is able to have both functions of recapture and deployment of the self-expanding prosthesis. Further, due to the protection of the outside external catheter sleeve-jointed on the outer side of the inside external catheter, risk of puncture of blood vessels due to contact between the inside external catheter and blood vessels when the inside external catheter passes through a three-dimensionally bending passage is able to be avoided.

In addition, the inside external catheter is manufactured to be a metal sheath which is partially hollow, so as to partially sacrifice the capacity of resistance to tension and compression to gain flexibility at a certain degree. Thus, the safe passing of the delivery device through the spatial three-dimensionally bending passage is able to be ensured, improving the safety of the whole device.

### Brief Description of the Drawings

FIG. 1 is a schematic structural diagram of a delivery device of a self-expanding prosthesis according to an embodiment of the present application;
FIG. 2 is a schematic sectional diagram of a catheter according to an embodiment of the present application;
FIG. 3 is a schematic sectional diagram of a handle according to an embodiment of the present application;
FIG. 4 is a schematic structural diagram of an internal catheter according to an embodiment of the present application;
FIG. 5 is a schematic sectional diagram of a fixed head of FIG. 4;
FIG. 6 is a schematic structural diagram of an inside external catheter according to an embodiment of the present application;
FIG. 7 is a schematic structural diagram of an outside external catheter on which a stabilizing tube is sleeve-jointed according to an embodiment of the present application;
FIG. 8 is a schematic structural diagram of a first threaded slider according to an embodiment of the present application;
FIG. 9a is a front view of a first locking part according to an embodiment of the present application;
FIG. 9b is a front view of a second locking part according to an embodiment of the present application;
FIG. 10 is a front view of the second locking part assembled with a second housing according to an embodiment of the present application;
FIG. 11 is a front view of the second locking part assembled between the second threaded knob and the second housing; and
FIG. 12 is a sectional diagram of FIG. 11.

In the figures:
1-Catheter;
   10-Internal catheter; 101-First elongated shaft; 102-Fixed head; 102a-Main body; 102b-Protrusion part; 102c-Guiding face;104-Second elongated shaft; 103-Conical head;
   20-Inside external catheter; 201-Third elongated shaft; 202-Metal sheath;
   30-Outside external catheter; 301-Fourth elongated shaft; 302-Polymer sheath;
   40-Stabilizing tube;
3-Self-expanding prosthesis;
5-Handle;
   51-Housing; 510-First housing; 511-Second housing; 512-Third housing;
   504-First threaded slider; 505-First threaded knob; 507-Guiding mechanism;
   506-First locking part; 506a, 503a-Button segment; 506b, 503b-Snap segment; 506c, 503c-Connection segment;
   501- Second threaded slider; 502-Second threaded knob; 503-Second locking part

### Detailed Description

A delivery device of a self-expanding prosthesis provided in the present application will be described in detail below with reference to the accompanying drawings and specific embodiments. Advantages and features of the present application will be more apparent from the description and appended claims. The accompanying drawings are provided in a very simplified form and not necessarily presented to exact scale, with the only intention to facilitate convenience and clarity in explaining the purpose of the present invention.

In the present application, the terms "proximal end" and "proximal side" refer to the end close to an operator, and accordingly the terms "distal end" and "distal side" refer to the end far away from the operator. For example, in the whole delivery device, a conical head is the farthest end of a catheter, and the connection of the catheter and a handle is the nearest end of the catheter.

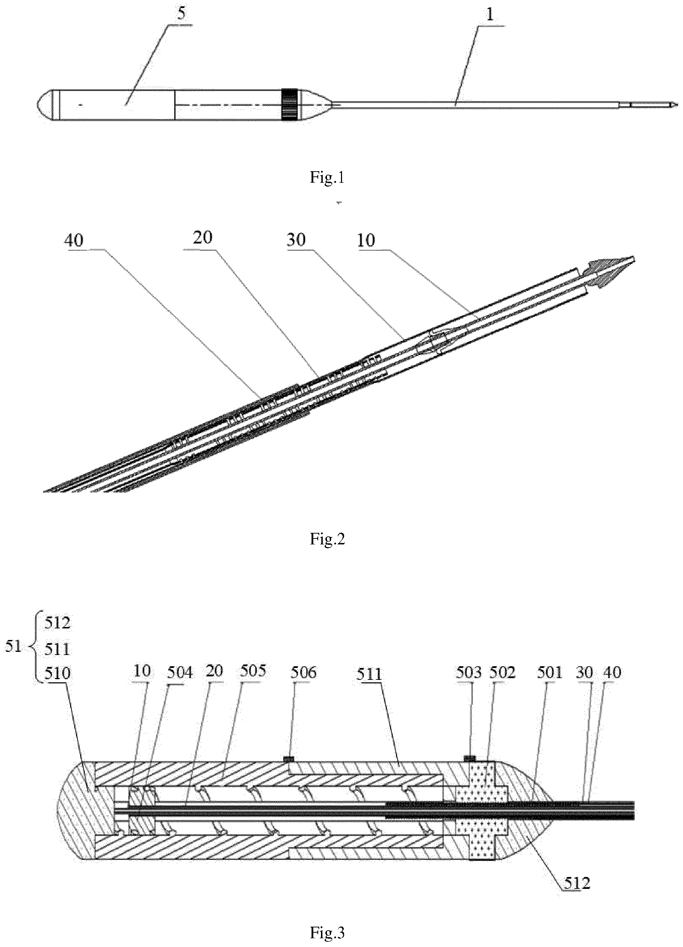

FIG. 1 is a schematic structural diagram of a delivery device of a self-expanding prosthesis in the present application. As shown in FIG. 1, the delivery device of a self-expanding prosthesis includes a catheter 1, and a handle 5 connected to the catheter 1. As shown in FIG. 2, the catheter 1 includes an internal catheter 10, an inside external catheter 20, an outside external catheter 30, and a stabilizing tube 40 that are sleeve-jointed in sequence. The states of the self-expanding prosthesis 3 include a compressed state, a semi-deployed state, and a deployed state. The internal catheter 10 is configured to accommodate the self-expanding prosthesis 3 in the compressed state. The inside external catheter 20 is able to move with respect to the internal catheter 10, and includes a distal end portion and a proximal end portion. The distal end portion is configured to drive the self-expanding prosthesis 3 to be compressed from the semi-deployed state to the compressed state. The outside external catheter 30 is able to move with respect to the internal catheter 10, and includes a distal end assembly and a proximal end assembly. The distal end assembly is used to restrain the self-expanding prosthesis 3 to maintain the compressed state of the self-expanding prosthesis 3. The distal end assembly has a better flexibility than the distal end portion, while the distal end portion has a higher tensile property and a higher compressive property than the distal end assembly. The stabilizing tube and the internal catheter stay still relative to each other.

Further, the proximal end of the internal catheter 10 is fixedly connected to the handle 5, and the stabilizing tube 40 is sleeve-jointed on the outer side of the external catheter 30 and is fixedly connected to the handle 5, so as to enhance stability and safety of the catheter 1 during recapture or deployment of the self-expanding prosthesis 3. The self-expanding prosthesis, for example, is a self-expanding heart valve prosthesis; and more specifically, is an aortic valve prosthesis, a mitral valve prosthesis, or a tricuspid valve prosthesis.

During use of the delivery device in this embodiment, the self-expanding prosthesis 3 may be deployed under cooperation of the handle 5, the internal catheter 10, and the outside external catheter 30. Under cooperation of the handle 5, the internal catheter 10, and the inside external catheter 20, the self-expanding prosthesis in a semi-deployed state may be compressed again and then recaptured, so as to facilitate the deployment again of the prosthesis. Thus, the prosthesis can be deployed at an optimal position, reducing the adverse effect caused by deviation of the deployed position. Specifically, when delivering the self-expanding prosthesis, the self-expanding prosthesis in a compressed state is loaded on the internal catheter 10 and restrained by the outside external catheter 30. When the self-expanding prosthesis in the compressed state is delivered near the target position, the proximal end assembly of the outside external catheter 30 moves with respect to the proximal end portion of the inside external catheter 20, so that the state of the self-expanding prosthesis is changed from the compressed state to the semi-deployed state. When the self-expanding prosthesis is delivered to the target position, the proximal end assembly of the outside external catheter 30 withdraws with respect to the proximal end portion of the inside external catheter 20, so that the self-expanding prosthesis in the semi-deployed state is further deployed until the self-expanding prosthesis is in a complete deployed state thereof.

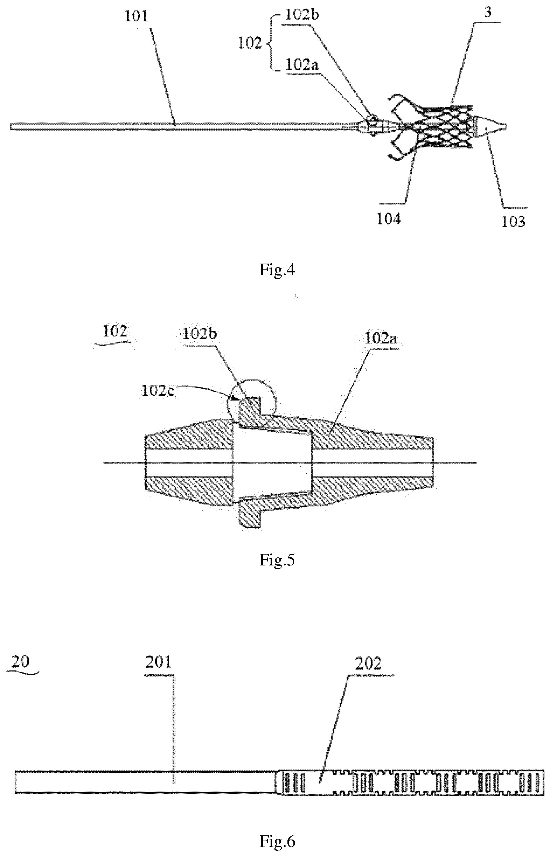

Referring to FIG. 4, which is a schematic structural diagram of the internal catheter 10 in an embodiment of the present application. The internal catheter 10 includes: a first elongated shaft 101, a fixed head 102, a second elongated shaft 104, and a conical head 103 that are successively connected. The end of the first elongated shaft 101 far away from the fixed head 102 is fixedly connected to the handle 5, and the first elongated shaft 101 is used for fixation and supporting. When delivering the self-expanding prosthesis 3, the self-expanding prosthesis 3 in the compressed state is loaded on the second elongated shaft 104, and the proximal end of the self-expanding prosthesis 3 is detachably connected to the fixed head 102.

As shown in FIG. 5, the fixed head 102 specifically includes a main body 102a and at least one protrusion part 102b. For example, when the self-expanding prosthesis is a heart valve prosthesis, the heart valve prosthesis includes an artificial heart valve stent, a valve, and a skirt. The artificial heart valve stent includes a stent main body and a lug connected to the stent main body. By hooking the lug to the protrusion part 102b, the artificial heart valve may be detachably connected to the fixed head 102. Each of the protrusion parts 102b is in an elastic connection to the main body 102a. Specifically, each protrusion part 102b is elastically connected to the main body 102a via an elastic structure, so that the protrusion part 102b can perform a telescopic motion along a radial direction of the main body 102to help in prevention of the unhooking caused by the self-expanding prosthesis when the inside external catheter 20 and the outside external catheter 30 move with respect to the internal catheter 10.

Moreover, in order to facilitate the smooth passage of the inside external catheter 20 through the fixed head to restrain the self-expanding prosthesis when the inside external catheter 20 moving towards the conical head 103, the outer side of the proximal end of each protrusion 102b is preferably provided with a guiding face 102c.

Referring to FIG. 6, which is a schematic structural diagram of an inside external catheter 20 in an embodiment of the present application. The proximal end portion of the inside external catheter 20 includes a third elongated shaft 201, and the distal end portion of the inside external catheter 20 includes a metal sheath 202. The third elongated shaft 201 is connected to the metal sheath 202. The end of the third elongated shaft 201 far away from the metal sheath 202 is connected to the handle 5, and can be move under the driving of the handle 5, so that the third elongated shaft 201 can move along the axial direction of the internal catheter 10. The third elongated shaft 201 is configured to be unrotatable along the radial direction of the third elongated shaft 201. Preferably, the third elongated shaft 201 is made of polymer or metal. Since the sheath made of metal having a high resistance to tension and compression, the self-expanding prosthesis may be radially compressed by the metal sheath 202. Therefore, the inside external catheter 20 of this embodiment meets the requirement of recapture of the self-expanding prosthesis. The present application does not particularly limit the metal material and a preparation method of the metal sheath 202, and any existing material and preparation method in the prior art can be used. Preferably, the metal sheath 202 is made of nitinol by weaving or cutting.

At least part of the metal sheath 202 has a hollow structure. Preferably, hollow portions in the hollow structure are in an alternative arrangement or a whorled arrangement, so that the metal sheath 202 is decreased in resistance to tension and compression but improved in the flexibility (i.e., sacrificing some capacity of the resistance to tension and compression to improve the flexibility). Herein, " alternative arrangement" should be understood that a plurality of rows of hollow portions are included in the axial direction, each row being comprised of a plurality of hollow repeat units that are circumferentially and uniformly distributed, the hollow repeat units of two adjacent rows arranged in a staggered manner, two adjacent hollow repeat units in the same line parallel to the axial direction being spaced by one row of hollow portions. Each hollow repeat unit may include one or more hollow elements. Herein, " whorled arrangement" should be understood that a plurality of rows of hollow portions are included in the axial direction, each row being at least comprised of one hollow repeat unit, each hollow repeat unit, in the axial projection, overlapping or forming an angle with the corresponding one of the hollow repeat units in the adjacent row. Each hollow repeat unit may include one or more hollow elements. In this way, the metal sheath 202 can get in or out of the passage with a small radius of curvature, such as the aortic arch, under the protection of the outside external catheter 30, so that risk of puncture of blood vessels due to contact between the inside external catheter 20 and blood vessels when the inside external catheter passes through a three-dimensionally bending passage is able to be avoided. Moreover, when acting on the self-expanding prosthesis, the metal sheath 202 of the inside external catheter 20 meets the property requirements in loading and recapturing the self-expanding prosthesis.

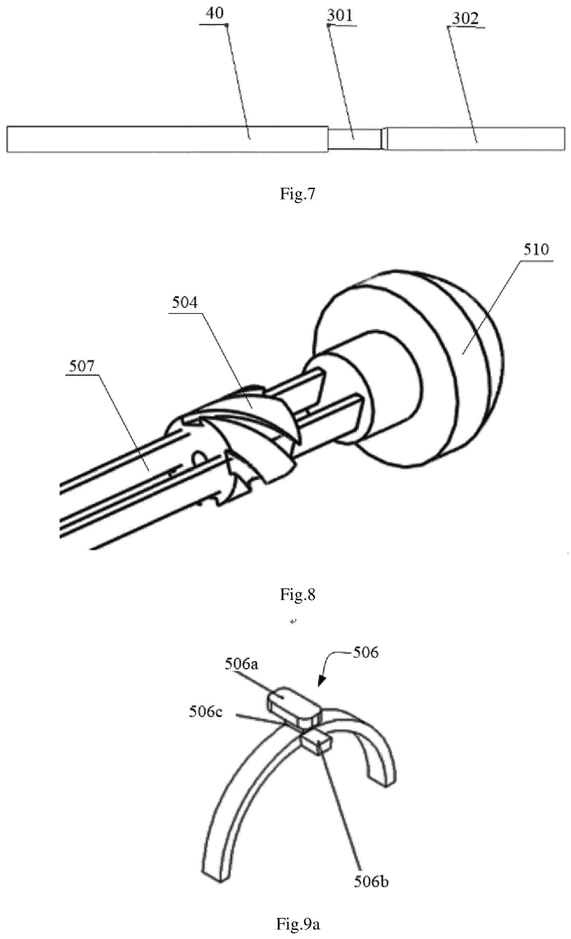

Referring to FIG. 7, which is a schematic structural diagram of the outside external catheter 30 on which the stabilizing tube 40 is sleeve-jointed according to an embodiment of the present application. The proximal end assembly of the outside external catheter 30 includes a fourth elongated shaft 301, and the distal end assembly of the outside external catheter 30 includes a polymer sheath 302. The fourth elongated shaft 301 is connected to the polymer sheath 302. The end of the fourth elongated shaft 301 far away from the polymer sheath 302 is connected to the handle 5, and is able to move under the driving of the handle 5 to move, so that the fourth elongated shaft 301 can move in the axial direction of the internal catheter 10. Moreover, the fourth elongated shaft 301 is configured to be unrotatable in the radial direction. The sheath made of polymer material has an excellent flexibility, particularly, a spatial three-dimensional bending capacity. Therefore, the outside external catheter 30 of this embodiment meets the property requirement during deployment of the self-expanding prosthesis. The present application does not particularly limit the polymer material and the preparation method of the polymer sheath 302, and any existing material and preparation method in the prior art can be used. Preferably, the polymer sheath 302 is made of PEBA, PA, PU, or TPE material by means of extrusion molding. The stabilizing tube 40 in this embodiment has functions similar to that in the prior art. For example, the stabilizing tube can prevent the external catheter from being directly exposed to the vessel and injuring the vessel during movement. Therefore, the stabilizing tube in this embodiment may be any existing stabilizing tube.

Referring to FIG. 3, which is a schematic sectional diagram of a handle according to an embodiment of the present application. The handle 5 includes a housing 51, and a first control mechanism and a second control mechanism that are located in the housing 51. The proximal end of the internal catheter 10 and the proximal end of the stabilizing tube 40 are both fixedly connected to the housing 51. The first control mechanism is configured to control movement of the inside external catheter 20, and the second control mechanism is configured to control movement of the outside external catheter 30. By cooperation of the first control mechanism and the second control mechanism, the state of the self-expanding prosthesis may be changed from a semi-deployed state to a compressed state, or from a compressed state to a semi-deployed state and then to a deployed state, thus simplifying the operation. To understand a specific control principle, reference is made to subsequent description about specific structures of the first control mechanism and the second control mechanism.

Referring to FIG. 3 continuously, the housing 51 includes a first housing 510, a second housing 511, and a third housing 512. The first control mechanism is located between the first housing 510 and the second housing 511, and the second control mechanism is located between the second housing 511 and the third housing 512. The proximal end of the internal catheter 10 is fixedly connected to the first housing 510, and the proximal end of the stabilizing tube 40 is fixedly connected to the third housing 512.

The first control mechanism includes a first threaded slider 504, a first threaded knob 505, and a first locking part 506. The first threaded knob 505 is restricted between the first housing 510 and the second housing 511, and is able to rotate about the axis of the handle (that is, the first threaded knob 505 is configured to be rotatable along the circumferential direction of the handle 5 and not movable along the axial direction of the handle 5). The first threaded slider 504 is configured to be fixedly connected to the inside external catheter 20, and is able to move along the axial direction of the handle 5 under the driving of the first threaded knob 505. The first locking part 506 is configured to limit rotation of the first threaded knob 505 (that is, the first locking part 506 can determine whether the first threaded knob 505 rotates or not).

The first threaded knob 505 has a first threaded cavity (the wall of the first threaded cavity is provided with inner threads), and the first threaded slider 504 is accommodated in the first threaded cavity and is connected to the threads of the first threaded knob 505 (that is, the first threaded slider 504 is provided with outer threads that match the inner threads on the wall of the first threaded cavity). The first threaded slider 504 is sleeve-jointed and fixed to the outer side of the inside external catheter 20 (specifically, the first threaded slider 504 is sleeve-jointed and fixed to the end of the third elongated shaft 201 far away from the metal sheath 202). The first locking part 506 is disposed between the first threaded knob 505 and the second housing 511, and is able to move forward or backward along the axial direction of the handle 5 to drive the first threaded knob 505 to be separated from and connected to the second housing 511. When the first threaded knob 505 is separated from the second housing 511, the first threaded knob 505 is able to rotate about the axial direction of the handle 5. When the first threaded knob 505 is connected to the second housing 511, the first threaded knob 505 and the second housing 511 amount to a whole (in this case, the first locking part 506 is used to immobilize the first threaded knob 505, which in turn brakes the first threaded slider 504 and no relative motion is existed therebetween.

Preferably, in order that the first threaded slider 504 can only move in the axial direction of the handle 5 but cannot rotate about the axis of the handle, the delivery device is further provided with a pair of guiding mechanisms 507 which are axially parallel to the axial direction of the handle 5. One end of each guiding mechanism 507 is connected to the first housing 510, and the other end of each guiding mechanism 507 extends through the first threaded slider 504 and then is fixedly connected to the second housing 511, to restrain rotation of the first threaded slider 504. As shown in FIG. 8, the first threaded slider 504 is axially provided with a first through hole, a second through hole, and a third through hole. The first through hole is a central hole. The inside external catheter 20 runs through the first through hole and is fixed on the first threaded slider 504, and the internal catheter 10 runs through the inside external catheter 20 and is fixed on the first housing 510.The two guiding mechanisms 507 run through the second through hole and the third through hole and are fixed on the first housing 510 and the second housing 511, respectively. In this way, the first threaded slider 504 can only move along the two guiding mechanisms 507, thus restraining rotation of the first threaded slider 504.

The first threaded slider 504 and the first threaded knob 505 are screwed to each other, and the first threaded slider 504 is sleeve-jointed and fixed to the outer side of the inside external catheter 20. Therefore, when the first threaded knob 505 is rotated clockwise or counterclockwise, the first threaded slider 504 is driven to move forward or backward (to move along the axial direction of the handle 5, converting the rotational motion to horizontal movement), which in turn drive the inside external catheter 20 to move forward or backward.

The second control mechanism includes a second threaded slider 501, a second threaded knob 502, and a second locking part 503. The second threaded knob 502 is restricted between the second housing 511 and the third housing 512, and can rotate along the circumference of the handle 5 (namely, about the axis of the handle) but cannot move axially.

The second threaded slider 501 is configured to be fixedly connected to the outside external catheter 30, and can move along the axial direction of the handle 5 under the driving of the second threaded knob 502. The second locking part 503 is configured to limit rotation of the second threaded knob 502.

The second threaded knob 502 has a second threaded cavity, and the inner wall of the second threaded cavity is provided with inner threads. After extending through the second threaded cavity, the second threaded slider 501 is connected to the outside external catheter 30. Moreover, the second threaded slider 501 is provided with outer threads, so as to form a threaded connection with the second threaded knob 502. Specifically, the outer wall of the second threaded slider 501 is provided with outer threads that match the inner threads on the inner wall of the second threaded cavity. The second threaded slider 501 is sleeve-jointed and fixed to the outer side of the proximal end of the fourth elongated shaft 301 of the outside external catheter 30, to form an assembly tube. Further, the assembly tube extends through the body cavity of the third housing 512, and then is accommodated in the stabilizing tube and extends to the distal end. The second locking part 503 is disposed between the second threaded knob 502 and the second housing 511, and can move forward or backward along the axial direction of the handle 5 to enable disconnection or connection between the second threaded knob 502 and the second housing 511. When the second threaded knob 502 and the second housing 511 are disconnected, the second threaded knob 502 is able to rotate about the axis of the handle 5. When the second threaded knob 502 and the second housing 511 are connected, the second threaded knob 502 and the second housing 511 amount to a whole (in this case, the second locking part 503 is used to immobilize the second threaded knob 502, which in turn brakes the second threaded slider 501 and no relative motion is existed therebetween.

Preferably, the inner wall of the third housing 512 is provided with rotation limiting grooves (not shown in the drawings) that are distributed along the axial direction of the housing, and the outer side of the second threaded slider 510 is provided with rotation limiting protrusions that fit into the rotation limiting grooves. The rotation limiting protrusions can move forward or backward along the rotation limiting grooves. Since the rotation limiting grooves are configured for limiting and guiding, the second threaded slider 501 can only move along the axial direction of the handle 5 but cannot rotate about the axis of the handle 5.

The second threaded slider 501 and the second threaded knob 502 are engaged with each other, and one end of the second threaded slider 501 is fixedly connected to the outside external catheter 30. Therefore, when the second threaded knob 502 is rotated clockwise or counterclockwise, the second threaded slider 501 is driven to move forward or backward (to move along the axial direction of the handle 5, converting the rotational motion to horizontal movement), which in turn drives the outside external catheter 30 to move forward or backward.

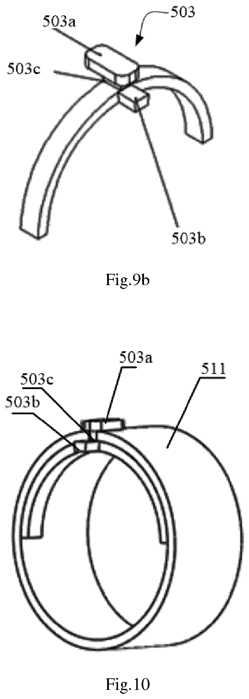

Referring to FIG. 9a and FIG. 9b, FIG. 9a is a front view of the first locking part in this embodiment and FIG. 9b is a front view of the second locking part in this embodiment.

The first locking part 506 includes a first button segment 506a, a first snap segment 506b, and a first connection segment 506c that connects the first button segment 506a and the first snap segment 506b. With reference to FIG. 3, the first button segment 506a is arranged on the outer side of the proximal end of the second housing 511, and is able to move along the axial direction of the second housing 511. The first connection segment 506c extends into the interior of the second housing 511 from the exterior of the second housing 511. The first snap segment 506b is located on the end surface of the first connection segment 506c close to the first threaded knob 505, and extends towards the first threaded knob 505 (in other words, the first snap segment 506b faces towards the end surface of the first threaded knob 505). The first button segment 506a is able to move along the axial direction of the second housing 511, to drive the first snap segment 506b to be separated from or connected to an adjacent first butting groove. Preferably, the first snap segment 506b is further provided with first stabilizing segments on two sides thereof. The stabilizing segment is arc-shaped to fit the inner wall of the proximal end of the second housing 511, so as to enhance stability of the first locking part during its axial movement.

The second locking part 503 is structurally similar to the first locking part 506, and includes a second button segment 503a, a second snap segment 503b, and a second connection segment 503c that connects the second button segment 503a and the second snap segment 503b. Their differences are as follows: The second button segment 503a is arranged on the outer side of the distal end of the second housing 511, and is able to move along the axial direction of the second housing 511. The second connection segment 503c extends into the interior of the second housing 511 from the exterior of the second housing 511. The second snap segment 503b is located on an end surface of the second connection segment 503c close to the second threaded knob 502, and extends towards the second threaded knob 502 (in other words, the second snap segment 503b faces towards the end surface of the second threaded knob 502).

In this embodiment, the end surface of the first threaded knob 505 adjacent to the first locking part 506 is circumferentially provided with a plurality of first butting grooves, and the end surface of the second threaded knob 502 adjacent to the second locking part 503 is circumferentially provided with a plurality of second butting grooves. Each of the first snap segment 506b and the second snap segment 503b fits into the butting grooves on the adjacent end surface.

Specifically, referring to FIG. 10, which is a front view of the second locking part 503 assembled with the second housing 511. The snap segment 503b faces away from the end surface of the second housing 511.

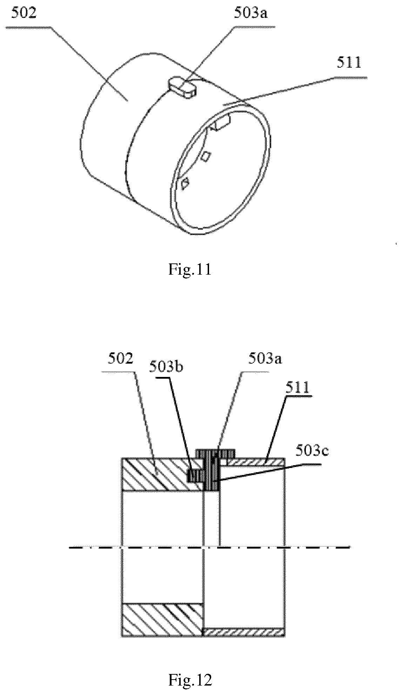

Referring to FIG. 11 and FIG. 12, FIG. 11 is a perspective view of the second locking part 503 assembled between the second threaded knob and the second housing, and FIG. 12 is a sectional diagram of FIG. 11. The second button segment 503a is arranged on the outer side of the distal end of the second housing 511, and is able to move along the axial direction of the second housing 511. The second connection segment 503c passes through a groove in the second housing 511 from the outer side of the second housing 511, and then radially extends to the inner side of the second housing 511. The second snap segment 503b is further provided with second stabilizing segments on two sides thereof. The stabilizing segment is arc-shaped to fit the inner wall of the distal end of the second housing 511. The second snap segment 503b is located on the end surface of the second connection segment 503c away from the second housing 511, and extends towards the second threaded knob 502. The second snap segment 503b fits into the second butting groove provided on the end surface of the second threaded knob 502.

To better understand the working principle of the delivery device of the self-expanding prosthesis in the present application, by taking the aortic valve prosthesis as an example, the loading, deployment, and recapture and re-deployment process of the aortic valve prosthesis is described in detail below with reference to FIG. 2, FIG. 4, FIG. 6 and FIG. 7.

Herein, the "compressed state" refers to the state in which the self-expanding prosthesis is radially compressed by an external radial force in order to allow the self-expanding prosthesis to smoothly pass through the tubular organs (for example, the blood vessels) of the human body to reach a target position.

The "deployed state" refers to the state in which the self-expanding prosthesis is expanded at the target position after reaching the target position and removing the external constraints.

The "semi-deployed state" refers to the state in which one part of the self-expanding prosthesis is no longer subjected to the radial restraint and starts to expand, while the other part of the self-expanding prosthesis is still restrained and compressed state.

The loading process of the aortic valve prosthesis is the process of compressing the aortic valve prosthesis from a deployed state to a compressed state.

The loading of the aortic valve prosthesis is performed with the aid of loading tools and other tools in an ice bath *in vitro.* Therefore, by use of the high-polymer sheath 302 in the outside external catheter 30 the aortic valve prosthesis in the compressed state may be well compressed and held on the second elongated shaft 104 that is between the fixed head 102 and the conical head 103. In this case, the metal sheath 202 is disposed on the rear side of the fixed head 102 (the metal sheath 202 does not cover the artificial heart valve).

The lug of the aortic valve prosthesis is fixed to the protrusion part 102b of the fixed head 102 by means of the loading tool, and the second threaded knob 502 is rotated in a positive direction (clockwise or counterclockwise). In this way, the polymer sheath 302 is pushed by the rotation of the second threaded knob 502, until the polymer sheath 302 completely covers the aortic valve prosthesis in the compressed state and attaches to the conical head 103. Thus, the loading of the aortic valve prosthesis is completed.

The deployment, and recapture and re-deployment process of the aortic valve prosthesis refers to the process of changing the valve prosthesis from the compressed state to a semi-deployed state/deployed state.

The core concept of the present application is as follows: In order to ensure that the sheath of the self-expanding prosthesis in a compressed state can smoothly pass through the arch in the high circuitous channel area such as the aortic arch, the sheath usually sacrifices the resistance to tension and compression to attain a good flexibility. When the self-expanding prosthesis changes into the semi-deployed state after being partially deployed in vivo, the self-expanding prosthesis needs to be recaptured and then compressed back to the compressed state due to an undesired deployment position or other reasons. In this case, the external catheter, especially the sheath, is required to have a large resistance to compression. It is difficult for general materials or structures of the pipe to balance the requirements between bending performance and resistance to tensile and compression. Therefore, it is necessary to use different properties at different time or in different space to resolve this contradiction.

Referring to FIG. 2, when the delivery device passes through the aortic arch, the aortic valve prosthesis in the compressed state is restrained by the polymer sheath 302 of the outside external catheter 30. In this case, the metal sheath 202 is located at the rear side of the fixed head 102. The polymer sheath 302 has a good flexibility and can be three-dimensionally curved, thereby ensuring that the delivery device is able to safely pass through the aortic arch.

After the aortic valve prosthesis reaches a targeted lesion position, the second threaded knob 502 is rotated in a negative direction (the direction opposite to the rotation direction of the artificial heart valve during loading), and the polymer sheath 302 is drawn back to deploy the aortic valve prosthesis. The aortic valve prosthesis is gradually deployed from an inflow tract, and is changed from the compressed state to a semi-deployed state. That is, one section (for example, the section in the outflow tract) of the aortic valve prosthesis is no longer subjected to the restraint and starts to expand, while the other section (for example, the section in the inflow tract) of the aortic valve prosthesis is still restrained and compressed by the polymer sheath 302. If an operator finds that the deployment position of the aortic valve prosthesis is undesired when the aortic valve prosthesis is partially deployed, there is a necessity to recapture the aortic valve prosthesis to adjust the deployment position. To recover the aortic valve prosthesis, the aortic valve prosthesis needs to be compressed from a semi-deployed state to a compressed state again, which needs to overcome a high resistance. Therefore, the metal sheath 202 having a high resistance to tension and compression is needed for recapture and compression of the prosthesis. The specific process is as follows:
Firstly, the second locking part 503 is pushed to brake the polymer sheath 302 of the outside external catheter 30. The first threaded knob 505 is rotated in the positive direction to allow the metal sheath 202 to be pushed towards the distal end under the rotation of the first threaded knob 505, until the aortic valve prosthesis in the semi-deployed state is compressed to the compressed state again. Despite a poor flexibility, the metal sheath 202 is protected by the outside external catheter 30 on its outer side. Therefore, risk of puncture of blood vessels due to contact between the metal sheath 202 and blood vessels when the metal sheath 202 passes through a three-dimensionally bending passage is able to be avoided. Moreover, since the protrusion part 102b of the fixed head 102 is elastically connected to the main body 102a of the fixed head 102, the protrusion part 102b is able to perform a telescopic motion along the radial direction of the main body 102a, so that the metal sheath 202 can smoothly pass through a place where the fixed head 102 adheres to the polymer sheath 302.

Afterwards, when the aortic valve prosthesis is entirely compressed and held in the metal sheath 202, the first locking part 506 is pushed to brake the metal sheath 202, and the brake of the polymer sheath 302 controlled by the second locking part 503 is removed. The second threaded knob 502 is rotated in the positive direction, and the polymer sheath 302 is pushed forward to the distal end so as to cover the whole aortic valve prosthesis.

Subsequently, the second locking part 503 is pushed to brake the polymer sheath 302, and the brake of the metal sheath 202 controlled by the first locking part 506 is removed. The first threaded knob 505 is rotated in the negative direction, and the metal sheath 202 is withdrawn towards the proximal end. Since the protrusion part 102b of the fixed head 102 is in a elastic connection to the main body 102a of the fixed head 102, the protrusion part 102b is able to perform a telescopic motion along the radial direction of the main body 102a, thereby ensuring the smooth passthrough of the metal sheath 202 without the occurrence of unhooking of the aortic valve prosthesis.

Finally, after the metal sheath 202 is successfully withdrawn, the first locking part 506 is pushed to brake the metal sheath 202. After an appropriate deployed position is determined, the brake of the polymer sheath 302 controlled by the second locking part 503 is removed, and then the aortic valve prosthesis can be repositioned and deployed again.

To sum up, in the delivery device of a self-expanding prosthesis provided in the present application, the delivery device of a self-expanding prosthesis includes a catheter and a handle connected to the catheter. The catheter includes an internal catheter, an inside external catheter, an outside external catheter, and a stabilizing tube that are sleeve-jointed one on another in sequence. The internal catheter is configured to accommodate the self-expanding prosthesis in a compressed state, and a distal end portion of the inside external catheter is configured to drive the self-expanding prosthesis to be compressed from a semi-deployed state to the compressed state. A distal end assembly of the outside external catheter is configured to contain the self-expanding prosthesis to maintain the compressed state of the self-expanding prosthesis. The distal end assembly has a better flexibility than the distal end portion, while the distal end portion has a higher tensile property and a higher compressive property than the distal end assembly. The stabilizing tube and the internal catheter remain stationary relative to each other. The self-expanding prosthesis is able to be deployed by means of the outside external catheter, and the self-expanding prosthesis in the semi-deployed state is able to be recompressed and recaptured to facilitate the re-deployment of prosthesis by means of the inside external catheter. Therefore, the single device of the present application is able to have both functions of recapture and deployment of the self-expanding prosthesis.

Further, due to the protection of the outside external catheter sleeve-jointed on the outer side of the inside external catheter, risk of puncture of blood vessels due to contact between the inside external catheter and blood vessels when the inside external catheter passes through a three-dimensionally bending passage is able to be avoided.

In addition, the inside external catheter is manufactured to be a metal sheath which is partially hollow, so as to partially sacrifice the capacity of resistance to tension and compression to gain a flexibility at a certain degree. Thus, the safe passing of the delivery device through the spatial three-dimensionally bending passage is able to be ensured, improving the safety of the whole device.

The above merely describes preferred embodiments of the present application, and is not intended to limit the scope of the present application.

In the foregoing embodiments, the outside external catheter is made of the pure polymer, and the inside external catheter is made of the pure metal. However, persons skilled in the art should understand that, the outside external catheter and/or the inside external catheter is not limited to being prepared from the foregoing material, and may be prepared by other appropriate materials, such as composite material. Specifically, the outside external catheter and/or the inside external catheter is prepared by weaving or twisting reinforcing ribs.

## Claims

1. A delivery device of a self-expanding prosthesis (3), comprising a catheter (1) and a handle (5) connected to the catheter (1), wherein the catheter (1) comprises an internal catheter (10), an inside external catheter (20), an outside external catheter (30), and a stabilizing tube (40) that are sleeve-jointed in sequence;
wherein states of the self-expanding prosthesis (3) include: a compressed state, a semi-deployed state, and a deployed state;
wherein the internal catheter (10) is configured to accommodate the self-expanding prosthesis (3) in the compressed state;
wherein the inside external catheter (20) is able to move with respect to the internal catheter (10), and comprises a distal end portion and a proximal end portion, the distal end portion configured to drive the self-expanding prosthesis (3) to be compressed from the semi-deployed state to the compressed state;
wherein the outside external catheter (30) is able to move with respect to the internal catheter (10), and comprises a distal end assembly and a proximal end assembly, the distal end assembly configured to contain the self-expanding prosthesis (3) to maintain the compressed state of the self-expanding prosthesis (3),
wherein when the self-expanding prosthesis (3) in the compressed state is delivered near a target position, the proximal end assembly of the outside external catheter (30) is configured to be moved with respect to the proximal end portion of the inside external catheter (20), so that a state of the self-expanding prosthesis is changed from the compressed state to the semi-deployed state and wherein when the self-expanding prosthesis is delivered to the target position, the proximal end assembly of the outside external catheter (30) is configured to be withdrawn with respect to the proximal end portion of the inside external catheter (20), so that the self-expanding prosthesis in the semi-deployed state is further deployed until the self-expanding prosthesis is in the deployed state;
wherein the distal end assembly of the outside external catheter (30) has a better flexibility than the distal end portion of the inside external catheter (20), while the distal end portion of the inside external catheter (20) has a higher tensile property and a higher compressive property than the distal end assembly of the outside external catheter (30); and wherein
the stabilizing tube (40) and the internal catheter (10) remain stationary relative to each other.

2. The delivery device of a self-expanding prosthesis (3) of claim 1, wherein the internal catheter (10) comprises, from a proximal end to a distal end, a first elongated shaft (101), a fixed head (102), a second elongated shaft (104), and a conical head (103) that are successively connected, wherein a proximal end of the first elongated shaft (101) is fixedly connected to the handle (5), and when delivering the self-expanding prosthesis (3), the self-expanding prosthesis (3) in a compressed state is loaded by the second elongated shaft (104), and is detachably connected to the fixed head (102) from a proximal end of the self-expanding prosthesis (3).

3. The delivery device of a self-expanding prosthesis (3) of claim 2, wherein the fixed head (102) comprises a main body (102a) and at least one protrusion part (102b), each protrusion part (102b) being in an elastic connection to the main body (102a).

4. The delivery device of a self-expanding prosthesis (3) of claim 3, wherein each protrusion part (102b) is connected to the main body (102a) via an elastic structure, so as to make the protrusion part (102b) capable of performing a telescopic motion along a radial direction of the main body (102a).

5. The delivery device of a self-expanding prosthesis (3) of claim 3, wherein a guiding face (102c) is provided at a proximal end of each protrusion part (102b) to facilitate a smooth passage of the inside external catheter (20).

6. The delivery device of a self-expanding prosthesis (3) of claim 1, wherein the proximal end portion of the inside external catheter (20) comprises a third elongated shaft (201), and the distal end portion of the inside external catheter (20) comprises a metal sheath (202), and wherein a proximal end of the third elongated shaft (201) is movably connected to the handle (5), and at least part of the metal sheath (202) has a hollow structure.

7. The delivery device of a self-expanding prosthesis (3) of claim 1, wherein the proximal end assembly of the outside external catheter (30) comprises a fourth elongated shaft (301), and the distal end assembly of the outside external catheter (30) comprises a polymer sheath (302), and wherein a proximal end of the fourth elongated shaft (301) is connected to the handle (5) and is movable under a driving of the handle (5).

8. The delivery device of a self-expanding prosthesis (3) of claim 1, wherein the handle (5) comprises a housing (51), and a first control mechanism and a second control mechanism that are located in the housing (51), wherein each of a proximal end of the internal catheter (10) and a proximal end of the stabilizing tube (40) is fixedly connected to the housing (51), and wherein the first control mechanism is configured to control movements of the inside external catheter (20), and the second control mechanism is configured to control movements of the outside external catheter (30).

9. The delivery device of a self-expanding prosthesis (3) of claim 8, wherein the housing (51) comprises a first housing (510), a second housing (511), and a third housing (512), wherein the first control mechanism is located between the first housing (510) and the second housing (511), and the second control mechanism is located between the second housing (511) and the third housing (512), and wherein the proximal end of the internal catheter (10) is fixedly connected to the first housing (510), and the proximal end of the stabilizing tube (40) is fixedly connected to the third housing (512).

10. The delivery device of a self-expanding prosthesis (3) of claim 9, wherein the first control mechanism comprises a first threaded slider (504), a first threaded knob (505), and a first locking part (506), wherein the first threaded knob (505) is restricted between the first housing (510) and the second housing (511) and is able to rotate about an axis of the handle (5), wherein the first threaded slider (504) is configured to be fixedly connected to the inside external catheter (20) and is able to move along an axial direction of the handle (5) under a driving of the first threaded knob (505) , and wherein the first locking part (506) is configured to control a rotation of the first threaded knob (505).

11. The delivery device of a self-expanding prosthesis (3) of claim 10, wherein the first threaded knob (505) has a first threaded cavity, into which the first threaded slider (504) is accommodated and is in a threaded connection with the first threaded knob (505), wherein the first threaded slider (504) is sleeve-jointed and fixed to an outer side of the inside external catheter (20), and wherein the first locking part (506) is disposed between the first threaded knob (505) and the second housing (511), and is able to move forward or backward along the axial direction of the handle (5) to drive the first threaded knob (505) to be separated from or connected to the second housing (511), preferably,
the first locking part (506) comprises a first button segment (506a), a first snap segment (506b), and a first connection segment (506c) that connects the first button segment (506a) and the first snap segment (506b), wherein the first button segment is arranged outside the second housing (511) and is able to move along an axial direction of the second housing (511), wherein the first connection segment (506c) extends into an interior of the second housing (511) from an exterior of the second housing (511), and wherein the first snap segment (506b) is located on an end surface of the first connection segment (506c) close to the first threaded knob (505), and extends towards the first threaded knob (505);
an end surface of the first threaded knob (505) adjacent to the first locking part (506) is provided with a first butting groove; and
the first snap segment (506b) fits into the first butting groove.

12. The delivery device of a self-expanding prosthesis (3) of claim 10, further comprising a pair of guiding mechanisms (507) which are axially parallel to the axial direction of the handle (5), wherein one end of each guiding mechanism (507) is connected to the first housing (510), and the other end of each guiding mechanism (507) extends through the first threaded slider (504) and is fixedly connected to the second housing (511), to restrain the rotation of the first threaded slider (504).

13. The delivery device of a self-expanding prosthesis (3) of claim 9, wherein the second control mechanism comprises a second threaded slider (501), a second threaded knob (502), and a second locking part (503), wherein the second threaded knob (502) is restricted between the second housing (511) and the third housing (512), and is able to rotate about an axis of the handle (5), wherein the second threaded slider (501) is configured to be fixedly connected to the outside external catheter (30), and is able to move along an axial direction of the handle (5) under a driving of the second threaded knob (502), and wherein the second locking part (503) is configured to limit rotation of the second threaded knob (502).

14. The delivery device of a self-expanding prosthesis (3) of claim 13, wherein the second threaded knob (502) has a second threaded cavity, wherein the second threaded slider (501) is connected to the outside external catheter (30) after extending through the second threaded cavity , and is provided with an outer thread to form a threaded connection with the second threaded knob (502), and wherein the second locking part (503) is disposed between the second threaded knob (502) and the second housing (511), and is able to move forward or backward along the axial direction of the handle (5) to drive the second threaded knob (502) to be separated from or connected to the second housing (511), preferably,
the second locking part (503) comprises a second button segment (503a), a second snap segment (503b), and a second connection segment (503c) that connects the second button segment (503a) and the second snap segment (503b), wherein the second button segment (503a) is arranged outside the second housing (511), and is able to move along an axial direction of the second housing (511), wherein the second connection segment (503c) extends into an interior of the second housing (511) from an exterior of the second housing (511) , and wherein the second snap segment (503b) is located on an end surface of the second connection segment (503c) close to the second threaded knob (502) and extends towards the second threaded knob (502);
an end surface of the second threaded knob (502) adjacent to the second locking part (503) is provided with a second butting groove; and
the second snap segment (503b) fits into the second butting groove.

15. The delivery device of a self-expanding prosthesis (3) of claim 13, wherein an inner wall of the third housing (512) is provided with rotation limiting grooves that are distributed along an axial direction of the housing (51), wherein an outer side of the second threaded slider (501) is provided with rotation limiting protrusions that fit into the rotation limiting grooves, and wherein the rotation limiting protrusions are able to move forward or backward along the rotation limiting grooves.

16. The delivery device of a self-expanding prosthesis (3) of any one of claims 1 to 15, wherein the self-expanding prosthesis (3) is an aortic valve prosthesis, a mitral valve prosthesis, or a tricuspid valve prosthesis.

## Patentansprüche

1. Zufuhrvorrichtung einer selbstexpandierenden Prothese (3), umfassend einen Katheter (1) und einen mit dem Katheter (1) verbundenen Griff (5), wobei
der Katheter (1) einen Innenkatheter (10), einen inneren Außenkatheter (20), einen äußeren Außenkatheter (30) und ein Stabilisierungsrohr (40) umfasst, die nacheinander mit einer Hülse verbunden sind;
wobei Zustände der selbstexpandierenden Prothese (3) Folgendes beinhalten: einen komprimierten Zustand, einen halb entfalteten Zustand und einen entfalteten Zustand;
wobei der Innenkatheter (10) konfiguriert ist, um die selbstexpandierende Prothese (3) in dem komprimierten Zustand aufzunehmen;
wobei der innere Außenkatheter (20) in der Lage ist, sich in Bezug auf den Innenkatheter (10) zu bewegen, und einen distalen Endabschnitt und einen proximalen Endabschnitt umfasst, wobei der distale Endabschnitt konfiguriert ist, um die selbstexpandierende Prothese (3) anzutreiben, um von dem halb entfalteten Zustand in den komprimierten Zustand komprimiert zu werden;
wobei der äußere Außenkatheter (30) in der Lage ist, sich in Bezug auf den internen Katheter (10) zu bewegen, und eine distale Endanordnung und eine proximale Endanordnung umfasst, wobei die distale Endanordnung konfiguriert ist, um die selbstexpandierende Prothese (3) zu enthalten, um den komprimierten Zustand der selbstexpandierenden Prothese (3) aufrechtzuerhalten,
wobei, wenn die selbstexpandierende Prothese (3) in dem komprimierten Zustand in die Nähe einer Zielposition zugeführt wird, die proximale Endanordnung des äußeren Außenkatheters (30) konfiguriert ist, um in Bezug auf den proximalen Endabschnitt des inneren Außenkatheters (20) bewegt zu werden, sodass ein Zustand der selbstexpandierenden Prothese von dem komprimierten Zustand in den halb entfalteten Zustand geändert wird, und wobei, wenn die selbstexpandierende Prothese zu der Zielposition gebracht wird, die proximale Endanordnung des äußeren Außenkatheters (30) konfiguriert ist, um in Bezug auf den proximalen Endabschnitt des inneren externen Katheters (20) zurückgezogen zu werden, sodass die selbstexpandierende Prothese in dem halb entfalteten Zustand weiter entfaltet wird, bis die selbstexpandierende Prothese in dem entfalteten Zustand ist;
wobei die distale Endanordnung des äußeren Außenkatheters (30) eine bessere Flexibilität aufweist als der distale Endabschnitt des inneren externen Katheters (20), während der distale Endabschnitt des inneren Außenkatheters (20) eine höhere Zugeigenschaft und eine höhere Druckeigenschaft aufweist als die distale Endanordnung des äußeren Außenkatheters (30); und wobei
das Stabilisierungsrohr (40) und der Innenkatheter (10) in Bezug aufeinander stationär bleiben.

2. Zufuhrvorrichtung einer selbstexpandierenden Prothese (3) nach Anspruch 1, wobei der Innenkatheter (10) von einem proximalen Ende zu einem distalen Ende einen ersten länglichen Schaft (101), einen feststehenden Kopf (102), einen zweiten länglichen Schaft (104) und einen konischen Kopf (103) umfasst, die nacheinander verbunden sind, wobei ein proximales Ende des ersten länglichen Schafts (101) fest mit dem Griff (5) verbunden ist und beim Zuführen der selbstexpandierenden Prothese (3) die selbstexpandierende Prothese (3) in einem komprimierten Zustand durch den zweiten länglichen Schaft (104) belastet wird und von einem proximalen Ende der selbstexpandierenden Prothese (3) lösbar mit dem feststehenden Kopf (102) verbunden ist.

3. Zufuhrvorrichtung einer selbstexpandierenden Prothese (3) nach Anspruch 2, wobei der feststehende Kopf (102) einen Hauptkörper (102a) und mindestens ein Vorsprungsteil (102b) umfasst, wobei jedes Vorsprungsteil (102b) in einer elastischen Verbindung mit dem Hauptkörper (102a) ist.

4. Zufuhrvorrichtung einer selbstexpandierenden Prothese (3) nach Anspruch 3, wobei jedes Vorsprungsteil (102b) mit dem Hauptkörper (102a) über eine elastische Struktur verbunden ist, um das Vorsprungsteil (102b) in die Lage zu versetzen, eine Teleskopbewegung entlang einer radialen Richtung des Hauptkörpers (102a) auszuführen.

5. Zufuhrvorrichtung einer selbstexpandierenden Prothese (3) nach Anspruch 3, wobei eine Führungsfläche (102c) an einem proximalen Ende von jedem Vorsprungsteil (102b) bereitgestellt ist, um einen reibungslosen Durchgang des inneren Außenkatheters (20) zu erleichtern.

6. Zufuhrvorrichtung einer selbstexpandierenden Prothese (3) nach Anspruch 1, wobei der proximale Endabschnitt des inneren Außenkatheters (20) einen dritten länglichen Schaft (201) umfasst, und der distale Endabschnitt des inneren Außenkatheters (20) eine Metallhülle (202) umfasst, und wobei ein proximales Ende des dritten länglichen Schafts (201) beweglich mit dem Griff (5) verbunden ist und zumindest ein Teil der Metallhülle (202) eine hohle Struktur aufweist.

7. Zufuhrvorrichtung einer selbstexpandierenden Prothese (3) nach Anspruch 1, wobei die proximale Endanordnung des äußeren Außenkatheters (30) einen vierten länglichen Schaft (301) umfasst und die distale Endanordnung des äußeren Außenkatheters (30) eine Polymerhülle (302) umfasst, und wobei ein proximales Ende des vierten länglichen Schafts (301) mit dem Griff (5) verbunden ist und bei einem Antrieb des Griffs (5) beweglich ist.

8. Zufuhrvorrichtung einer selbstexpandierenden Prothese (3) nach Anspruch 1, wobei der Griff (5) ein Gehäuse (51) und einen ersten Steuermechanismus und einen zweiten Steuermechanismus umfasst, die sich in dem Gehäuse (51) befinden, wobei sowohl ein proximales Ende des Innenkatheters (10) als auch ein proximales Ende des Stabilisierungsrohrs (40) fest mit dem Gehäuse (51) verbunden ist, und wobei der erste Steuermechanismus konfiguriert ist, um Bewegungen des inneren Außenkatheters (20) zu steuern, und der zweite Steuermechanismus konfiguriert ist, um Bewegungen des äußeren Außenkatheters (30) zu steuern.

9. Zufuhrvorrichtung einer selbstexpandierenden Prothese (3) nach Anspruch 8, wobei das Gehäuse (51) ein erstes Gehäuse (510), ein zweites Gehäuse (511) und ein drittes Gehäuse (512) umfasst, wobei sich der erste Steuermechanismus zwischen dem ersten Gehäuse (510) und dem zweiten Gehäuse (511) befindet, und sich der zweite Steuermechanismus zwischen dem zweiten Gehäuse (511) und dem dritten Gehäuse (512) befindet, und wobei das proximale Ende des Innenkatheters (10) fest mit dem ersten Gehäuse (510) verbunden ist und das proximale Ende des Stabilisierungsrohrs (40) fest mit dem dritten Gehäuse (512) verbunden ist.

10. Zufuhrvorrichtung einer selbstexpandierenden Prothese (3) nach Anspruch 9, wobei der erste Steuermechanismus einen ersten Gewindeschieber (504), einen ersten Gewindeknopf (505) und ein erstes Verriegelungsteil (506) umfasst, wobei der erste Gewindeknopf (505) zwischen dem ersten Gehäuse (510) und dem zweiten Gehäuse (511) eingeschränkt ist und in der Lage ist, um eine Achse des Griffs (5) zu drehen, wobei der erste Gewindeschieber (504) konfiguriert ist, um fest mit dem inneren Außenkatheter (20) verbunden zu sein und in der Lage ist, sich bei Antrieb des ersten Gewindeknopfs (505) entlang einer axialen Richtung des Griffs (5) zu bewegen, und wobei das erste Verriegelungsteil (506) konfiguriert ist, um eine Drehung des ersten Gewindeknopfs (505) zu steuern.

11. Zufuhrvorrichtung einer selbstexpandierenden Prothese (3) nach Anspruch 10, wobei der erste Gewindeknopf (505) einen ersten Gewindehohlraum aufweist, in dem der erste Gewindeschieber (504) aufgenommen ist und in einer Schraubverbindung mit dem ersten Gewindeknopf (505) ist, wobei der erste Gewindeschieber (504) mit einer Hülse verbunden und an einer Außenseite des inneren Außenkatheters (20) befestigt ist, und wobei das erste Verriegelungsteil (506) zwischen dem ersten Gewindeknopf (505) und dem zweiten Gehäuse (511) angeordnet ist und in der Lage ist, sich entlang der axialen Richtung des Griffs (5) vorwärts oder rückwärts zu bewegen, um den ersten Gewindeknopf (505) anzutreiben, um von dem zweiten Gehäuse (511) getrennt oder damit verbunden zu werden, vorzugsweise,
das erste Verriegelungsteil (506) ein erstes Knopfsegment (506a), ein erstes Rastsegment (506b) und ein erstes Verbindungssegment (506c) umfasst, das das erste Knopfsegment (506a) und das erste Rastsegment (506b) verbindet, wobei das erste Knopfsegment außerhalb des zweiten Gehäuses (511) angeordnet ist und sich entlang einer axialen Richtung des zweiten Gehäuses (511) bewegen kann, wobei sich das erste Verbindungssegment (506c) von einer Außenseite des zweiten Gehäuses (511) in ein Inneres des zweiten Gehäuses (511) erstreckt, und wobei das erste Rastsegment (506b) an einer Endfläche des ersten Verbindungssegments (506c) nahe dem ersten Gewindeknopf (505) angeordnet ist und sich zu dem ersten Gewindeknopf (505) hin erstreckt;
eine Endfläche des ersten Gewindeknopfs (505), die an den ersten Verriegelungsteil (506) angrenzt, mit einer ersten Anschlagnut versehen ist; und
das erste Rastsegment (506b) in die erste Anschlagnut passt.

12. Zufuhrvorrichtung einer selbstexpandierenden Prothese (3) nach Anspruch 10, ferner umfassend ein Paar Führungsmechanismen (507), die axial parallel zu der axialen Richtung des Griffs (5) sind, wobei ein Ende von jedem Führungsmechanismus (507) mit dem ersten Gehäuse (510) verbunden ist und sich das andere Ende von jedem Führungsmechanismus (507) durch den ersten Gewindeschieber (504) erstreckt und fest mit dem zweiten Gehäuse (511) verbunden ist, um die Drehung des ersten Gewindeschiebers (504) zu begrenzen.

13. Zufuhrvorrichtung einer selbstexpandierenden Prothese (3) nach Anspruch 9, wobei der zweite Steuermechanismus einen zweiten Gewindeschieber (501), einen zweiten Gewindeknopf (502) und ein zweites Verriegelungsteil (503) umfasst, wobei der zweite Gewindeknopf (502) zwischen dem zweiten Gehäuse (511) und dem dritten Gehäuse (512) eingeschränkt ist und in der Lage ist, um um eine Achse des Griffs (5) zu drehen, wobei der zweite Gewindeschieber (501) konfiguriert ist, um fest mit dem äußeren Außenkatheter (30) verbunden zu sein, und in der Lage ist, sich bei einem Antrieb des zweiten Gewindeknopfs (502) entlang einer axialen Richtung des Griffs (5) zu bewegen, und wobei das zweite Verriegelungsteil (503) konfiguriert ist, um eine Drehung des zweiten Gewindeknopfs (502) zu begrenzen.

14. Zufuhrvorrichtung einer selbstexpandierenden Prothese (3) nach Anspruch 13, wobei der zweite Gewindeknopf (502) einen zweiten Gewindehohlraum aufweist, wobei der zweite Gewindeschieber (501) mit dem äußeren Außenkatheter (30) verbunden ist, nachdem er sich durch den zweiten Gewindehohlraum erstreckt hat, und mit einem Außengewinde versehen ist, um eine Schraubverbindung mit dem zweiten Gewindeknopf (502) zu bilden, und wobei das zweite Verriegelungsteil (503) zwischen dem zweiten Gewindeknopf (502) und dem zweiten Gehäuse (511) angeordnet ist und in der Lage ist, sich entlang der axialen Richtung des Griffs (5) vorwärts oder rückwärts zu bewegen, um den zweiten Gewindeknopf (502) von dem zweiten Gehäuse (511) zu trennen oder damit zu verbinden, vorzugsweise
das zweite Verriegelungsteil (503) ein zweites Knopfsegment (503a), ein zweites Rastsegment (503b) und ein zweites Verbindungssegment (503c), das das zweite Knopfsegment (503a) und das zweite Rastsegment (503b) verbindet, umfasst, wobei das zweite Knopfsegment (503a) außerhalb des zweiten Gehäuses (511) angeordnet ist und in der Lage ist, sich entlang einer axialen Richtung des zweiten Gehäuses (511) zu bewegen, wobei sich das zweite Verbindungssegment (503c) von einer Außenseite des zweiten Gehäuses (511) in ein Inneres des zweiten Gehäuses (511) erstreckt, und wobei das zweite Rastsegment (503b) an einer Endfläche des zweiten Verbindungssegments (503c) nahe dem zweiten Gewindeknopf (502) angeordnet ist und sich zu dem zweiten Gewindeknopf (502) hin erstreckt;
eine Endfläche des zweiten Gewindeknopfs (502) angrenzend an das zweite Verriegelungsteil (503), mit einer zweiten Anschlagnut versehen ist; und
das zweite Rastsegment (503b) in die zweite Anschlagnut passt.

15. Zufuhrvorrichtung einer selbstexpandierenden Prothese (3) nach Anspruch 13, wobei eine Innenwand des dritten Gehäuses (512) mit Drehbegrenzungsnuten versehen ist, die entlang einer axialen Richtung des Gehäuses (51) verteilt sind, wobei eine Außenseite des zweiten Gewindeschiebers (501) mit Drehbegrenzungsvorsprüngen versehen ist, die in die Drehbegrenzungsnuten passen, und wobei die Drehbegrenzungsvorsprünge in der Lage sind, sich entlang der Drehbegrenzungsnuten vorwärts oder rückwärts zu bewegen.

16. Zufuhrvorrichtung einer selbstexpandierenden Prothese (3) nach einem der Ansprüche 1 bis 15, wobei die selbstexpandierende Prothese (3) eine Aortenklappenprothese, eine Mitralklappenprothese oder eine Trikuspidalklappenprothese ist.

## Revendications

1. Dispositif de pose d'une prothèse auto-expansible (3), comprenant un cathéter (1) et une poignée (5) reliée au cathéter (1), dans lequel
le cathéter (1) comprend un cathéter interne (10), un cathéter externe intérieur (20), un cathéter externe extérieur (30) et un tube de stabilisation (40) qui sont reliés par manchon dans l'ordre ;
dans lequel les états de la prothèse auto-expansible (3) comprennent : un état comprimé, un état semi-déployé et un état déployé ;
dans lequel le cathéter interne (10) est configuré pour accueillir la prothèse auto-expansible (3) à l'état comprimé ;
dans lequel le cathéter externe intérieur (20) peut se déplacer par rapport au cathéter interne (10) et comprend une partie d'extrémité distale et une partie d'extrémité proximale, la partie d'extrémité distale étant configurée pour entraîner la compression de la prothèse auto-expansible (3) de l'état semi-déployé à l'état comprimé ;
dans lequel le cathéter externe extérieur (30) peut se déplacer par rapport au cathéter interne (10), et comprend un ensemble d'extrémité distale et un ensemble d'extrémité proximale, l'ensemble d'extrémité distale étant configuré pour contenir la prothèse auto-expansible (3) afin de maintenir l'état comprimé de la prothèse auto-expansible (3),
dans lequel, lorsque la prothèse auto-expansible (3) à l'état comprimé est délivrée près d'une position cible, l'ensemble d'extrémité proximale du cathéter externe extérieur (30) est configuré pour être déplacé par rapport à la partie d'extrémité proximale du cathéter externe intérieur (20), de sorte qu'un état de la prothèse auto-expansible passe de l'état comprimé à l'état semi-déployé et dans lequel, lorsque la prothèse auto-expansible est délivrée à la position cible, l'ensemble d'extrémité proximale du cathéter externe extérieur (30) est configuré pour être retiré par rapport à la partie d'extrémité proximale du cathéter externe intérieur (20), de sorte que la prothèse auto-expansible à l'état semi-déployé est encore déployée jusqu'à ce que la prothèse auto-expansible se trouve dans l'état déployé ;
dans lequel l'ensemble d'extrémité distale du cathéter externe extérieur (30) présente une meilleure flexibilité que la partie d'extrémité distale du cathéter externe intérieur (20), tandis que la partie d'extrémité distale du cathéter externe intérieur (20) présente une propriété de traction supérieure et une propriété de compression supérieure à celles de l'ensemble d'extrémité distale du cathéter externe extérieur (30) ; et dans lequel
le tube de stabilisation (40) et le cathéter interne (10) restent fixes l'un par rapport à l'autre.

2. Dispositif de pose d'une prothèse auto-expansible (3) selon la revendication 1, dans lequel le cathéter interne (10) comprend, d'une extrémité proximale à une extrémité distale, une première tige allongée (101), une tête fixe (102), une deuxième tige allongée (104), et une tête conique (103) qui sont connectées successivement, dans lequel une extrémité proximale de la première tige allongée (101) est reliée de manière fixe à la poignée (5), et lors de la pose de la prothèse auto-expansible (3), la prothèse auto-expansible (3) à l'état comprimé est chargée par la deuxième tige allongée (104), et est reliée de manière amovible à la tête fixe (102) à partir d'une extrémité proximale de la prothèse auto-expansible (3).

3. Dispositif de pose d'une prothèse auto-expansible (3) selon la revendication 2, dans lequel la tête fixe (102) comprend un corps principal (102a) et au moins une partie en saillie (102b), chaque partie en saillie (102b) étant en liaison élastique avec le corps principal (102a).

4. Dispositif de pose d'une prothèse auto-expansible (3) selon la revendication 3, dans lequel chaque partie en saillie (102b) est reliée au corps principal (102a) par une structure élastique, de façon à rendre la partie en saillie (102b) capable d'effectuer un mouvement télescopique le long d'une direction radiale du corps principal (102a).

5. Dispositif de pose d'une prothèse auto-expansible (3) selon la revendication 3, dans lequel une face de guidage (102c) est fournie à une extrémité proximale de chaque partie en saillie (102b) pour faciliter un passage en douceur du cathéter externe intérieur (20).

6. Dispositif de pose d'une prothèse auto-expansible (3) selon la revendication 1, dans lequel la partie d'extrémité proximale du cathéter externe intérieur (20) comprend une troisième tige allongée (201), et la partie d'extrémité distale du cathéter externe intérieur (20) comprend une gaine métallique (202), et dans lequel une extrémité proximale de la troisième tige allongée (201) est connectée de manière mobile à la poignée (5), et au moins une partie de la gaine métallique (202) a une structure creuse.

7. Dispositif de pose d'une prothèse auto-expansible (3) selon la revendication 1, dans lequel l'ensemble d'extrémité proximale du cathéter externe extérieur (30) comprend une quatrième tige allongée (301), et l'ensemble d'extrémité distale du cathéter externe extérieur (30) comprend une gaine en polymère (302), et dans lequel une extrémité proximale de la quatrième tige allongée (301) est reliée à la poignée (5) et est mobile sous l'action de la poignée (5).

8. Dispositif de pose d'une prothèse auto-expansible (3) selon la revendication 1, dans lequel la poignée (5) comprend un logement (51), et un premier mécanisme de commande et un second mécanisme de commande qui sont situés dans le logement (51), dans lequel chacun d'une extrémité proximale du cathéter interne (10) et d'une extrémité proximale du tube de stabilisation (40) est relié de manière fixe au logement (51), et dans lequel le premier mécanisme de commande est configuré pour commander les mouvements du cathéter externe intérieur (20), et le second mécanisme de commande est configuré pour commander les mouvements du cathéter externe extérieur (30).

9. Dispositif de pose d'une prothèse auto-expansible (3) selon la revendication 8, dans lequel le logement (51) comprend un premier logement (510), un deuxième logement (511), et un troisième logement (512), dans lequel le premier mécanisme de commande est situé entre le premier logement (510) et le deuxième logement (511), et le second mécanisme de commande est situé entre le deuxième logement (511) et le troisième logement (512), et dans lequel l'extrémité proximale du cathéter interne (10) est reliée de manière fixe au premier logement (510), et l'extrémité proximale du tube de stabilisation (40) est reliée de manière fixe au troisième logement (512).

10. Dispositif de pose d'une prothèse auto-expansible (3) selon la revendication 9, dans lequel le premier mécanisme de commande comprend un premier coulisseau fileté (504), un premier bouton fileté (505), et une première partie de verrouillage (506), dans lequel le premier bouton fileté (505) est restreint entre le premier logement (510) et le deuxième logement (511) et peut tourner autour d'un axe de la poignée (5), dans lequel le premier coulisseau fileté (504) est configuré pour être relié de manière fixe au cathéter externe intérieur (20) et peut se déplacer le long d'une direction axiale de la poignée (5) sous l'action du premier bouton fileté (505), et dans lequel la première partie de verrouillage (506) est configurée pour commander une rotation du premier bouton fileté (505).

11. Dispositif de pose d'une prothèse auto-expansible (3) selon la revendication 10, dans lequel le premier bouton fileté (505) a une première cavité filetée, dans laquelle le premier coulisseau fileté (504) est logé et est en connexion filetée avec le premier bouton fileté (505), dans lequel le premier coulisseau fileté (504) est assemblé par manchon et fixé à un côté extérieur du cathéter externe intérieur (20), et dans lequel la première partie de verrouillage (506) est disposée entre le premier bouton fileté (505) et le deuxième logement (511), et peut se déplacer vers l'avant ou vers l'arrière le long de la direction axiale de la poignée (5) pour entraîner le premier bouton fileté (505) afin qu'il soit séparé du ou relié au deuxième logement (511), de préférence,
la première partie de verrouillage (506) comprend un premier segment de bouton (506a), un premier segment d'encliquetage (506b), et un premier segment de connexion (506c) qui relie le premier segment de bouton (506a) et le premier segment d'encliquetage (506b), dans lequel le premier segment de bouton est disposé à l'extérieur du deuxième logement (511) et peut se déplacer le long d'une direction axiale du deuxième logement (511), dans lequel le premier segment de connexion (506c) s'étend à l'intérieur du deuxième logement (511) depuis l'extérieur du deuxième logement (511), et dans lequel le premier segment d'encliquetage (506b) est situé sur une surface d'extrémité du premier segment de connexion (506c) à proximité du premier bouton fileté (505), et s'étend vers le premier bouton fileté (505) ;
une surface d'extrémité du premier bouton fileté (505) adjacente à la première partie de verrouillage (506) est pourvue d'une première rainure d'aboutement ; et
le premier segment d'encliquetage (506b) s'insère dans la première rainure d'aboutement.

12. Dispositif de pose d'une prothèse auto-expansible (3) selon la revendication 10, comprenant en outre une paire de mécanismes de guidage (507) qui sont axialement parallèles à la direction axiale de la poignée (5), dans lequel une extrémité de chaque mécanisme de guidage (507) est reliée au premier logement (510), et l'autre extrémité de chaque mécanisme de guidage (507) s'étend à travers le premier coulisseau fileté (504) et est reliée de manière fixe au deuxième logement (511), pour restreindre la rotation du premier coulisseau fileté (504).

13. Dispositif de pose d'une prothèse auto-expansible (3) selon la revendication 9, dans lequel le second mécanisme de commande comprend un second coulisseau fileté (501), un second bouton fileté (502), et une seconde partie de verrouillage (503), dans lequel le second bouton fileté (502) est restreint entre le deuxième logement (511) et le troisième logement (512) et peut tourner autour d'un axe de la poignée (5), dans lequel le second coulisseau fileté (501) est configuré pour être relié de manière fixe au cathéter externe extérieur (30) et peut se déplacer le long d'une direction axiale de la poignée (5) sous l'action du second bouton fileté (502), et dans lequel la seconde partie de verrouillage (503) est configurée pour limiter la rotation du second bouton fileté (502).

14. Dispositif de pose d'une prothèse auto-expansible (3) selon la revendication 13, dans lequel le second bouton fileté (502) a une seconde cavité filetée, dans lequel le second coulisseau fileté (501) est connecté au cathéter externe extérieur (30) après s'être étendu à travers la seconde cavité filetée, et est pourvu d'un filetage externe pour former une connexion filetée avec le second bouton fileté (502), et dans lequel la seconde partie de verrouillage (503) est disposée entre le second bouton fileté (502) et le deuxième logement (511), et peut se déplacer vers l'avant ou vers l'arrière le long de la direction axiale de la poignée (5) pour entraîner le second bouton fileté (502) afin qu'il soit séparé du ou relié au deuxième logement (511), de préférence,
la seconde partie de verrouillage (503) comprend un second segment de bouton (503a), un second segment d'encliquetage (503b), et un second segment de connexion (503c) qui relie le second segment de bouton (503a) et le second segment d'encliquetage (503b), dans lequel le second segment de bouton (503a) est disposé à l'extérieur du deuxième logement (511) et peut se déplacer le long d'une direction axiale du deuxième logement (511), dans lequel le second segment de connexion (503c) s'étend à l'intérieur du deuxième logement (511) depuis l'extérieur du deuxième logement (511), et dans lequel le second segment d'encliquetage (503b) est situé sur une surface d'extrémité du second segment de connexion (503c) à proximité du second bouton fileté (502), et s'étend vers le second bouton fileté (502) ;
une surface d'extrémité du second bouton fileté (502) adjacente à la seconde partie de verrouillage (503) est pourvue d'une seconde rainure d'aboutement ; et
le second segment d'encliquetage (503b) s'insère dans la seconde rainure d'aboutement.

15. Dispositif de pose d'une prothèse auto-expansible (3) selon la revendication 13, dans lequel une paroi intérieure du troisième logement (512) est pourvue de rainures limitant la rotation qui sont réparties le long d'une direction axiale du logement (51), dans lequel un côté extérieur du second coulisseau fileté (501) est pourvu de saillies limitant la rotation qui s'insèrent dans les rainures limitant la rotation, et dans lequel les saillies limitant la rotation sont capables de se déplacer vers l'avant ou vers l'arrière le long des rainures limitant la rotation.

16. Dispositif de pose d'une prothèse auto-expansible (3) selon l'une quelconque des revendications 1 à 15, dans lequel la prothèse auto-expansible (3) est une prothèse de valve aortique, une prothèse de valve mitrale ou une prothèse de valve tricuspide.
